# NOUVEAU FASCICULE DE BREVET EUROPEEN

(11) **EP 0 443 912 B2**
(45) Date de publication et mention de la décision concernant l'opposition: **04.06.1997**
(45) Mention de la délivrance du brevet: 01.06.1994
(21) Numéro de dépôt: 91400354.6
(22) Date de dépôt: 12.02.1991
(51) Int. Cl.: C07C 43/04, C07C 19/08, C09K 3/30, C08J 9/16, C08J 9/06

(54) **Mélanges de dimethyléther et de 1,1,1,2-tétrafluoroéthane et leurs applications**
Gemische von Dimethyläther und 1,1,1,2-Tetrafluoräthan und deren Verwendungen
Mixtures of dimethylether and 1,1,1,2-tetrafluoroethane and their applications

(30) Priorité: 20.02.1990 FR 9002012
(43) Date de publication de la demande: 28.08.1991
(73) Titulaire: ELF ATOCHEM S.A., 92800 Puteaux (FR)
(72) Inventeur: Arnaud, Didier, F-92400 Courbevoie (FR); Tanguy, Jean-Claude, F-95110 Sannois (FR)
(74) Mandataire: Leboulenger, Jean

(56) Documents cités:
- EP-A- 0 030 127
- DE-C- 2 737 132
- US-A- 2 885 427
- US-A- 3 607 755
- US-A- 4 129 603
- US-A- 4 755 316
- US-A- 4 771 080
- US-A- 4 783 276
- US-A- 4 810 316
- WORLD PATENT INDEX DATABASE, no. d'accession 78-29937A, semaine 16, 1978, Derwent Publications Ltd., London, GB
- CHEMICAL ABSTRACTS, vol. 87, no. 25, 19 décembre 1977, Columbus, Ohio, US; ANON (DU PONT): "Fluorcarbon azeotropes", page 660; colonne de gauche; ref. no. 200710Q
- AEROSOL AGE, May 1982, Roundtable Discussion: Du Pont talks about its DME propellant, pp.20, 24

## Description

La présente invention concerne des mélanges de fluides frigorigènes à bas point d'ébullition, n'ayant pas d'action sur l'ozone stratosphérique et utilisables pour remplacer les chlorofluorocarbures (CFC) dans les systèmes de réfrigération, en particulier domestique, à compression.

Il est maintenant établi qu'à cause de leur coefficient important d'action sur l'ozone, les CFC devront, à longue échéance, être remplacés par des fluides frigorigènes ne contenant pas de chlore et, de ce fait, n'ayant pas d'action sur l'ozone stratosphérique.

Le 1,1,1,2-tétrafluoroéthane (HFA 134a) a déjà été proposé comme substitut au dichlorodifluorométhane (CFC 12). Cependant, compte tenu de ses performances thermodynamiques plus faibles que celles du CFC 12 (en particulier son coefficient de performance), le HFA 134a ne constitue pas une solution pleinement satisfaisante si l'on veut minimiser les coûts d'exploitation d'une installation frigorifique ; ceci est particulièrement le cas des réfrigérateurs domestiques à compression qui utilisent actuellement comme fluide frigorigène le CFC 12 et pour lesquels les constructeurs sont soumis à des contraintes de consommations énergétiques de plus en plus fortes.

Le terme "coefficient de performance" (COP) désigne, ici et dans ce qui suit, le rapport de la capacité frigorifique du fluide frigorigène sur le travail de compression théorique nécessaire pour comprimer les vapeurs de fluide frigorigène. De ce coefficient de performance relatif au fluide dépend directement le coefficient de performance de l'installation.

Il a maintenant été trouvé que les mélanges de diméthyléther (DME) et de 1,1,1,2-tétrafluoréthane (HFA 134a) présentent des coefficients de performance plus importants que le HFA 134a pur. En particulier, il a été trouvé que, pour des fractions massiques de HFA 134a comprises entre 5 % et 65 % et de DME comprises entre 35 % et 95 %, le mélange présente un comportement pseudo-azéotropique conduisant à un gain substantiel et exceptionnellement stable du coefficient de performance.

Il a également été trouvé que le DME et le HFA 134a forment un azéotrope à point d'ébullition maximal égal à environ -22,4°C à 1,013 bar et dont la teneur en HFA 134a au point d'ébullition normal est d'environ 62,3 % en masse. Bien entendu, cette composition varie en fonction de la pression du mélange et, à une pression donnée, peut être facilement déterminée suivant des techniques bien connues.

Du fait de la valeur élevée de leurs coefficients de performance, on utilisera avantageusement comme fluides frigorigènes les mélanges contenant en masse d'environ 5 % à 85 % de HFA 134a (de préférence de 5 à 65 % environ) et d'environ 15 à 95 % de DME (de préférence de 35 à 95 % environ). Un mélange frigorigène tout particulièrement préféré est l'azéotrope décrit plus haut

Compte tenu de leurs propriétés physiques proches de celles des CFC, les mélanges selon l'invention peuvent également être utilisés comme propulseurs d'aérosol ou comme agents d'expansion des mousses plastiques.

Les exemples suivants illustrent l'invention, sans la limiter.

### EXEMPLE 1

L'azéotrope selon l'invention a été étudié expérimentalement à différentes températures par analyse, en chromatographie phase gaz, des compositions de la phase liquide et de la phase vapeur pour différents mélanges de HFA 134a et de DME.

Les pressions ont été mesurées avec une précision supérieure à 0,02 bar au moyen d'un manomètre HEI-SE. Les températures ont été mesurées à 0,02°C près au moyen d'une sonde de platine 1000 ohms

Le graphe 1 en annexe représente la courbe d'équilibre liquide/vapeur des mélanges HFA 134a/DME, établie à la température de 20,2°C. Sur ce graphe, l'axe des abscisses indique la fraction massique en HFA 134a et l'axe des ordonnées la pression absolue en bar; les signes □ correspondent aux points expérimentaux.

Pour chaque température on obtient une courbe analogue à celle du graphe 1. Par ajouts successifs de HFA 134a dans le DME, la pression développée par le mélange diminue régulièrement, puis passe par un mininum et croît régulièrement, ce qui met en évidence l'existence de l'azéotrope à point d'ébullition maximal.

La corrélation des points expérimentaux ainsi obtenus pour plusieurs isothermes a été effectuée selon des techniques bien connues, au moyen d'une simulation informatique.

Les points normaux d'ébullition ainsi déterminés pour différentes compositions en HFA 134a, sont résumés dans le tableau 1 suivant :

**TABLEAU 1**

| Composition massique en HFA 134a % | Point d'ébullition normal °C |
|---|---|
| 100 | - 25,80 |
| 90 | - 24,12 |
| 80 | - 23,04 |
| 70 | - 22,51 |
| 60 | - 22,41 |
| 62,3 | - 22,40 |
| 50 | - 22,60 |
| 40 | - 22,97 |
| 30 | - 23,43 |
| 20 | - 23,92 |
| 10 | - 24,40 |
| 0 | - 24,85 |

Les résultats de ces corrélations mettent en évidence le maximum du point d'ébullition normal pour une fraction massique du HFA 134a égale à 62,3 % ; ce qui permet de caractériser l'azéotrope par:
. son point d'ébullition normal qui est égal à environ -22,4°C
. sa composition massique en HFA 134a égale à environ 62,3 %

Le tableau 2 suivant donne la relation pression/température pour cet azéotrope, comparée à celle des corps purs

**TABLEAU 2**

| Température (°C) | Pression absolue (bar) | | |
|---|---|---|---|
| | Azéotrope DME/HFA 134a | HFA 134a pur | DME pur |
| - 20,0 | 1,12 | 1,31 | 1,24 |
| 5,8 | 3,08 | 3,62 | 3,28 |
| 20,2 | 4,96 | 5,79 | 5,20 |
| 50,0 | 11,50 | 13,40 | 11,70 |

La tension de vapeur de l'azéotrope reste sur une large gamme de température inférieure à la tension de vapeur des corps purs ; ces données indiquent que le mélange reste azéotropique dans tout cet intervalle de température.

D'autre part, pour des compositions massiques en HFA 134a variant entre 5 et 65 %, la tension de vapeur du mélange reste exceptionnellement stable (variation inférieure à 4 %). Ceci met en évidence le comportement pseudo-azéotropique de ce mélange sur cette gamme de composition.

### EXEMPLE 2

Cet exemple illustre l'utilisation des mélanges selon l'invention comme fluides frigorigènes. Les coefficients de performance des mélanges selon l'invention ont été comparés aux coefficients de performance des deux constituants seuls, pour un cycle thermodynamique standard défini comme suit :
. température de condensation : + 30°C
. température d'évaporation : - 20°C
. sous-refroidissement liquide : + 0°C
. surchauffe des vapeurs : + 0°C

Le graphe 2 illustre ces résultats. L'axe des abscisses indique la fraction massique en HFA 134a et l'axe des ordonnées la valeur du COP.

On peut observer que pour des fractions massiques en HFA 134a inférieures à 65 %, le COP du mélange frigorigène reste exceptionnellement stable, supérieur de 45 % à celui du HFA 134a.

Le tableau 3 suivant compare, pour le méme cycle, les coefficients de performance de l'azéotrope selon l'invention avec ceux du HFA 134a pur et du CFC 12 pur.

**TABLEAU 3**

| | COP |
|---|---|
| CFC 12 | 4,03 |
| HFA 134a | 3,97 |
| Azéotrope DME/HFA 134a | 4,14 |

On peut observer que le mélange azéotropique (outre l'avantage qu'il présente par rapport au HFA 134a pur) présente également une valeur de COP supérieure de 3 % (pour le cycle étudié ici), par rapport à la valeur de COP du CFC 12 pur.

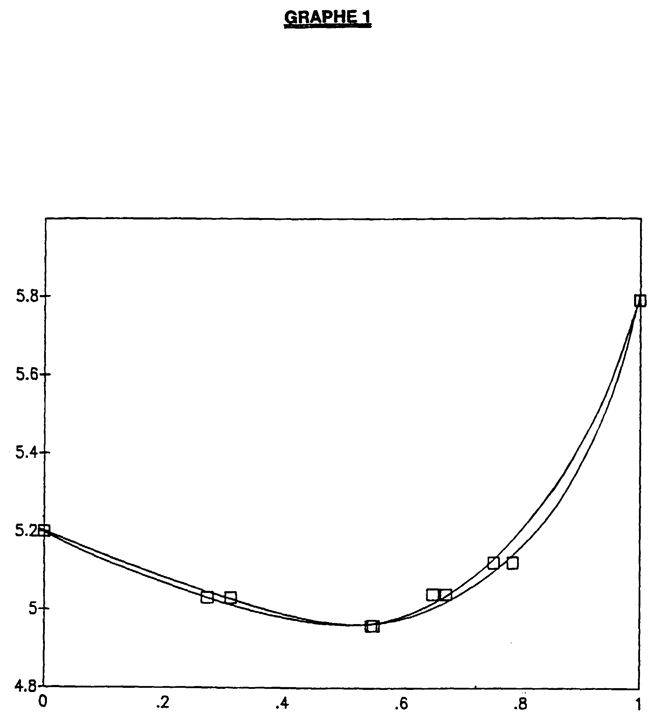

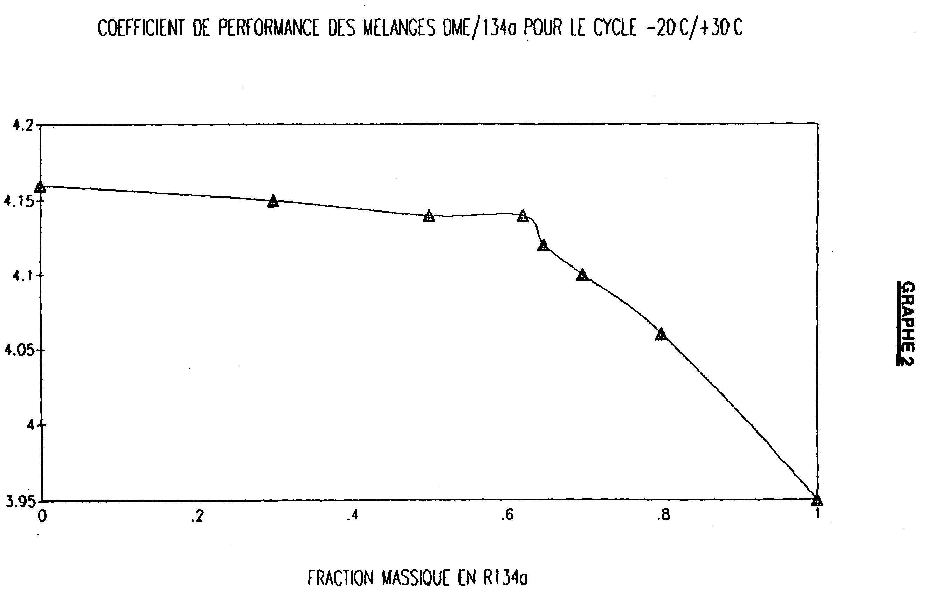

## Revendications

1. Mélange contenant en masse 5 à 65 % de 1,1,1,2-tétrafluoroéthane et 35 à 95 % de diméthyléther.

2. Mélange selon la revendication 1, caractérisé en ce qu'il contient environ 62,3 % en masse de 1,1,1,2-tétrafluoroéthane et 37,7 % en masse de diméthyléther et correspond à un mélange azéotropique à point d'ébullition maximal (environ -22,4°C sous 1,013 bar).

3. L'utilisation d'un mélange selon la revendication 1 ou 2 comme fluide frigorigène.

4. L'utilisation d'un mélange selon la revendication 1 ou 2 comme propulseur d'aérosols.

5. L'utilisation d'un mélange selon la revendication 1 ou 2 comme agent d'expansion des mousses plastiques.

## Patentansprüche

1. Gemisch, enthaltend 5 bis 65 Masse-% 1,1,1,2-Tetrafluoroethan und 35 bis 95 % Dimethylether.

2. Gemisch nach Anspruch 1, dadurch gekennzeichnet, daß es etwa 62.3 Masse-% 1,1,1,2-Tetrafluoroethan und 37,7 Masse-% Dimethylether enthält und einer azeotropen Mischung mit hohem Siedepunkt entspricht (ungefähr -22,4°C bei 1,013 bar).

3. Verwendung eines Gemischs nach Anspruch 1 oder 2 als Kältemittel.

4. Verwendung eines Gemischs nach Anspruch 1 oder 2 als Treibgas.

5. Verwendung eines Gemischs nach Anspruch 1 oder 2 als Schwellmittel für Plastikschaum.

## Claims

1. Mixture containing, on a mass basis, 5 to 65 % of 1,1,1,2-tetrafluoroethane and 35 to 95 % of dimethyl ether.

2. Mixture according to claim 1, characterized in that it contains approximately 62.3 mass% of 1,1,1,2-tetrafluoroethane and 37.7 mass % of dimethyl ether and corresponds to an azeotropic mixture with a maximum boiling point (approximately -22.4°C at 1.013 bar).

3. Use of a mixture according to claim 1 or 2 as refrigerant fluid.

4. Use of a mixture according to claim 1 or 2 as aerosol propellant.

5. Use of a mixture according to claim 1 or 2 as blowing agent for plastic foams.
